# EUROPEAN PATENT SPECIFICATION

(11) **EP 1 962 836 B1**
(45) Date of publication and mention of the grant of the patent: **29.12.2010**
(21) Application number: 06837161.6
(22) Date of filing: 07.11.2006
(51) Int. Cl.: A61K 31/4168, A61K 9/06

(54) **TREATMENT OF LENGTH DEPENDENT NEUROPATHY**
BEHANDLUNG VON LÄNGENABHÄNGIGER NEUROPATHIE
TRAITEMENT DE NEUROPHATHIES LONGUEUR-DEPENDANTES

(30) Priority: 08.11.2005 US 734423 P
(43) Date of publication of application: 03.09.2008
(60) Divisional application: 10173217.0
(73) Proprietor: Arcion Therapeutics, Inc., Baltimore, MD 21224 (US)
(72) Inventor: CAMPBELL, James, N., Luthersville, MD 21093 (US)
(74) Representative: HOFFMANN EITLE
(86) International application number: PCT/US2006/043499
(87) International publication number: WO 2007/056460

(56) References cited:
- WO-A-92/14453
- WO-A-2007/025613
- US-A- 6 054 461
- US-A- 6 147 102
- D. ZEIGLER, S. A. LYNCH, J. MUIR, J. BENJAMIN, M. B. MAX: "Transdermal clonidine versus placebo in painful diabetic neuropathy" PAIN, vol. 48, 1992, pages 403-408, XP002435596

## Description

This application claims priority under 35 U.S.C. § 119 to U.S.S.N. 60/734,423 filed November 8, 2005 by James N. Campbell.

### FIELD OF THE INVENTION

The invention is directed to clonidine and dosage formulations comprising the same for use in the treatment of pain associated with length dependent neuropathies that result in pain, which is not sympathetically maintained pain such as may result from diabetes and other conditions.

### BACKGROUND OF THE INVENTION

Pain often develops from diseases that affect the somatosensory system. One disease that is often implicated is diabetes mellitus. Diabetes may affect the nervous system in different ways but one of the classical disorders is a length dependent neuropathy. Here axons with longer axons preferentially are involved in a neuropathy which is associated with both degeneration and a sensitization of nociceptors. The classic feature is burning pain typically involving the feet given that the axons to the feet represent the longest primary afferents in the body. This problem may occur early or late in the disease, and in fact may occur in so-called pre-diabetes which is a condition representing a disorder of glucose metabolism without strictly meeting the criteria for diabetes mellitus. It is now appreciated that diabetes is but one cause of a length dependent neuropathy. The painful symptoms that accompany these disorders, including an idiopathic small fiber neuropathy, are nearly identical with that seen in diabetes mellitus. Treatments directed at treatment of the diabetes mellitus itself may help slow the progression of the neuropathy but do not necessarily address the pain. There are no known treatments for idiopathic length dependent small fiber neuropathy. Certain chemotherapeutic drugs induce a length dependent neuropathy associated with pain. This pain may limit dosing and thus affect the adequacy of the cancer treatment. Systemic treatments of pain include use of opioids, anticonvulsants, antidepressants, and membrane stabilizers. All of these therapies are frequently ineffective and typically their use is accompanied by a substantial adverse side effect profile. Systemic therapies can be given by the oral route, or by patches applied to the skin. Lidocaine patches can be applied to the skin. Their value in treatment of pain associated with length dependent neuropathies is limited because of numbing of the skin. Capsaicin can be applied locally to the skin but application is associated with significant pain and the capsaicin destroys nociceptor function.

Some prior attempts also have been made to treat painful diabetic neuropathy with clonidine, a potent α₂ -adrenergic partial agonist used primarily for the treatment of hypertension (Jarrott et al., "Clonidine: Understanding its disposition, sites, and mechanism of action", Clin. Exp. Pharm. Physiol., 14, 471-479 (1987)). Clonidine has been applied topically to areas remote to the painful area as an alternative to oral delivery for effecting systemic delivery. For example, in a placebo-controlled cross-over pain trial in patients with painful diabetic neuropathy, no statistically significant difference between patients receiving systemic clonidine administered with transdermal patches and patients receiving placebo patches was observed (Zeigler et al. Pain 48: 403-408 (1992)). In a follow-up placebo controlled pain study in similar patients with painful diabetic neuropathy, transdermal patches delivering systemic levels of clonidine were evaluated using a two-stage enriched enrollment design (Byas-Smith et al. Pain 60: 267-274 (1995)). Only twelve of forty-one patients (29%) who completed the initial course of treatment were considered clonidine responders. These twelve clonidine responders were then rechallenged in a second placebo controlled study which used the highest dosage available with the transdermal patch system. The pain reduction relative to placebo tended to be modest although statistically significant (p<0.015). The site of action of clonidine was not studied in this study. In principal the site of action could be central or peripheral. In other pain conditions a central analgesic action of clonidine has been determined. This treatment involved systemic delivery of clonidine with a transdermal patch applied remote to the painful area, resulting in systemic blood levels exceeding 0.2 ng/ml.

Further documents concerned with the treatment of pain are US 6,147,102 A, WO 92/14453 A, US 6,054,461 A, and WO 2007/025613 A. Said latter document was published after the filing date of the present patent (application), but claims priority and filing dates which are earlier than the priority and filing dates of the present patent (application), respectively.

It is therefore an object of the present invention to provide methods and compositions to effectively treat or alleviate pain in length dependent or other neuropathies, as may be associated with diabetes, by topical local delivery to the painful area of an alpha-2 adrenergic agonist.

### BRIEF SUMMARY OF THE INVENTION

Clonidine and compositions comprising the same are provided for the treatment of pain due to length dependent neuropathy by local or topical delivery of concentrations of the alpha₂ adrenergic agonist clonidine, to the painful area, without producing systemically effective levels of the clonidine. The compounds are delivered to or adjacent to painful areas in patients with length dependent neuropathy that results in pain associated with disease or damage to the pain signaling primary afferent (sensory) fibers and their receptor, not sympathetically maintained pain. For example, in a patient with painful diabetic neuropathy where the complaint is burning pain in the feet the alpha-2 agonist is topically applied to the feet in the painful region. A compound for the treatment of patients with diabetic neuropathy is clonidine applied in an ointment, gel, lotion, or transdermal patch, wherein the dosage is sufficient to provide an effective dose in the painful area or immediately adjacent areas, without producing pharmacologically active systemic blood levels.

### BRIEF DESCRIPTION OF THE DRAWINGS

Figure 1 is a graph of pain score over time (1-9 weeks) that shows the mean NGPS reduction by week, diamond, 0.1% clonidine; square, 0.2% clonidine, and circle, placebo.
Figure 2 is a graph of mean clonidine plasma concentratations over time from first dose (in days) of 0.1% clonidin for treatment A (dark circles): 3.15 g/day (3.1 mg clonidine HCl) and treatment B (opcn circles) 6.23 g/day (6.2 mg clonidine HCl).

### DETAILED DESCRIPTION OF THE INVENTION

### I. Formulations

### A. α₂-adrenergic agonist

The treatment or reduction of the symptoms (i.e. burning, pain) associated with length dependent neuropathies includes locally or topically administering an effective amount of an α₂-adrenergic agonist or combination thereof α₂-adrenergic agonists are known to those skilled in the art. See, for example, The Pharmacological Basis of Therapeutics, 8th Edition, Gill, A. G., T. W. Rall, A. S. Nies, P. Taylor, editors (Pergamon Press, Co., Inc., NY 1990).

According to the present invention, the α2 adrenergic agonist to be used is clonidine.

Although described above with reference specific to the compound, one can also utilize salts of the active compound. Methods for synthesis of this compound are known to those skilled in the art. Examples of pharmaceutically acceptable salts include, but are not limited to, mineral or organic acid salts of basic residues such as amines . The pharmaceutically acceptable salts include the conventional non-toxic salts or the quaternary ammonium salts of the parent compound formed, for example, from non-toxic inorganic or organic acids. Conventional non toxic salts include those derived from inorganic acids such as hydrochloric, hydrobromic, sulfuric, sulfamic, phosphoric and nitric acid; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, tolunesulfonic, methanesulfonic, ethane disulfonic, oxalic and isethionic acids. The pharmaceutically acceptable salts can be synthesized from the parent compound, which contains a basic moiety, by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed. (Mack Publishing Company, Easton, PA, 1985, p. 1418).

### B. Excipients

- These compounds have previously been administered systemically (either orally, skin patch, or by injection). Systemic administration either does not work or requires high doses and is thus associated with systemic side effects such as fatigue, dizziness, tiredness, headache, constipation, nausea, vomiting, diarrhea, insomnia, and dry mouth. Topical administration is described for treatment of sympathetically maintained pain in U.S. Patent No. 5,447,947 issued September 5, 1995 to Campbell, and in U.S. Patent Nos. 6,534,048 issued March 18, 2003 to Borgman and 6,147,102 issued November 15, 2000 to Borgman.
- In the method described herein, the compounds are administered locally or topically directly to or adjacent the painful area, in a suitable pharmaceutical carrier, many of which are known to those skilled in the art. The carder can be in the form of a lotion, ointment, gel, solution, or transdermal patch. Topical administration also includes iontophoresis wherein an electric current drives the drug, in the form of an ion such as a pharmaceutically acceptable salt, into the skin. The topical application allows the drug to reach high concentration at the painful area or tissue immediately adjacent thereto, avoiding many of the side effects of these compounds observed following systemic administration.

Formulation of drugs is discussed in, for example, Hoover, John E., Remington's Pharmaceutical Sciences, Mack Publishing Co., Easton, Pennsylvania (1975), and Liberman, H.A. and Lachman, L., Eds., Pharmaceutical Dosage Forms, Marcel Decker, New York, N.Y. (1980). The active compounds (or pharmaceutically acceptable salts thereof) may be administered per se or in the form of a pharmaceutical composition wherein the active compound(s) is in admixture or mixture with one or more pharmaceutically acceptable carriers, excipients or diluents. Pharmaceutical compositions may be formulated in conventional manner using one or more physiologically acceptable carriers comprising excipients and auxiliaries which facilitate processing of the active compounds into preparations which can be used pharmaceutically.

### C. Combination Therapies

The dosage formulation can be administered alone or in combination with a therapy such as an opioid, anticonvulsant, membrane stabilizer, and/or psychoactive drugs (for example, anti-depressants).

These may be formulated with the agonist in the pharmaceutically acceptable carrier for topical or local administration or administered in a separate formulation, with the other therapy being administered locally (by subcutaneous or intramuscular injection), topically or systemically, to the patient.

### II. Methods of Administration

### A. Patients to be Treated

A variety of diseases can affect the peripheral nervous system. Many of these disorders are not painful, but if the pain signaling system is affected, then pain may result. The prototype painful neuropathy stems from diabetes.

The most common effects on the nervous system result from a length dependent neuropathy. This means that the longer the sensory axon the more likely the axon may be affected. Given that the axons that go to the feet are the longest primary afferents in the body, these fibers are affected first. As the disease progresses, other axons shorter in length are affected. The length dependent neuropathies may be caused by a variety of diseases. The most common (60-70%) is diabetes. These neuropathies may also be caused by kidney disease, hormonal imbalances, vitamin deficiencies, alcoholism, autoimmune disorders, toxins, chemotherapy, and infections (e.g., AIDS).

In a preferred embodiment, the α2 adrenergic agonist compound and/or dosage formulation are for use in the treatment of patients as specified in the appended claims 1 and 9 that are patients with neuropathy that stems from diabetes mellitus. In another preferred embodiment, the α2 adrenergic agonist compound and/or dosage formulation are for use in the treatment of patients as specified in the appended claims 1 and 9 that are patients with a sensory peripheral neuropathy in the painful region. In yet another embodiment, the α2 adrenergic agonist compound and/or dosage formulation are for use in the treatment of patients as specified in the appended claims 1 and 9 that are patients with a small fiber neuropathy in the painful region.

### B. Dosages and Treatment Regimes

The dosage formulation is administered from once a week to several times a day, depending on the patient. In one embodiment, the therapeutic agent clonidine is administered in a concentration between 0.1 and 10% clonidine. The dose is determined by the region of pain. Because the effect of the clonidine is local it must be applied to the painful area. Thus in patients with broader areas of pain a higher dose of clonidine will be necessary though the percent concentration remains constant. The area treated is constrained by the systemic dosing. When 0.5% clonidine is applied to both feet, systemic effects may emerge as blood levels will approach those observed in treatment of hypertension. In the study done with 0.1% and 0.2% clonidine described below, the mean blood level was well below 0.1 ng/mg (one third of patients had no detectable clonidine in the blood), whereas the blood levels exceed 0.2 ng/ml with systemic delivery.

### Example 1: Formulation of Nenclon^{™} (clonidine) 0.1% Topical Gel

Neuclon™ (clonidine) 0.1% Topical Gel contains 0.1 mg of clonidine hydrochloride per 1.0 gram gel. The gel is formulated at pH 8.0 in order to maximize the amount of clonidine freebase in the product. Clonidine hydrochloride has a pKa of 8.2. The formulation is shown in Table 1.

**Table 1. Formulation of Neuclon^{™} (clonidine) 0.1% Topical Gel**

| Ingredient | % (w/w) |
|---|---|
| Cloniditte hydrochloride USP | 0.1% |
| Benzyl alcohol NF | 1.0% |
| Carbopol 980 NF | 0.6% |
| Sodium hydroxide NF | adjust to pH 8 |
| Hydrochloric acid NF | adjust to pH 8 (if necessary) |
| Purified water USP | qs ad 100% |

### Example 2: Treatment of patients with painful diabetic neuropathy

The objective of this study was to test the analgesic effects of 0.1% and 0.2% topical clonidine gel compared to a placebo gel in patients with chronic, lower extremity, painful diabetic neuropathy.

### Materials and Methods

One hundred sixty-six (166) adult patients with chronic, lower extremity, painful diabetic neuropathy who met the study admission criteria were enrolled in this multicenter study. Diabetic patients with a clinical diagnosis of neuropathy pain had to have an average daily pain score of 5 or greater on the 11-point Numerical Graphic Pain Scale (NGPS). Patients were allowed to continue using other medications for neuropathic pain, provided dosing with these medications was unchanged for 30 days prior to enrolment and during the study.

Patients were randomized to either 0.1% or 0.2% topical clonidine gel or placebo gel for this blinded, parallel design, 10-week study. Drug treatment lasted for 8 weeks, beginning with twice a day dosing for two weeks and escalating to three times a day for the remaining six weeks.

Analgesic efficacy was assessed on a weekly basis during the first month and biweekly during the second month by use of the following assessment instruments: Numerical Graphic Pain Scale (NGPS), Pain Relief Scale, Allodynia Scales for touching and cooling, Patient and Investigator Global Improvement assessments and Functional Interference Scales. Patients maintained daily outcome assessment diaries for the duration of the study.

Patient demographics for all three treatment groups were similar, as shown in Table 2.

**Table 2. Patient demographics for all treatment groups**

| **Group** | **Ago in Years (Range)** | **Gender** | **Diabetes Duration in Years (Range)** | **Diabetes Type II** | **Race*** | **Pain Duration in Years (Range)** |
|---|---|---|---|---|---|---|
| 0.1% | 61.2 (35-84) | 46.3 % F | 13.3 (1-44) | 83.3% | 83.3% C, 9.3% B, 7.4% H | 5.5 (1-16) |
| 0.2% | 61.0 (30-84) | 55.6% F | 12.4 (1-44) | 84.2% | 77.8% C, 3.7% H 14.8% H 3.7% O | 5.6(1-21) |
| Placebo | 61.2 (37-83) | 54.4%F | 10.5 (1.37) | 84.2% | 82.4 % C, 8.8% B, 8.8% H | 4.4(1-20) |

| | | | | | | |
|---|---|---|---|---|---|---|
| *C = Caucasian, B = Back, H = Hispanic, O = Other | | | | | | |

A total of 14 patients dropped out of the study: 8 on 0.1% gel, 5 on 0.2% gel, and 1 on placebo. Results of a 3 factor (Investigator, Time and Treatment) repeated measures analysis of variance revealed no significant differences with respect to Time (p=1.00) or Investigator (p=0.598), and no significant interaction between Time and Treatment (p=0.817) and Treatment and Investigator (p=0.805).

### Results

Patients randomized to 0.1% clonidine gel had a significantly greater reduction in NGPS averaged over time, compared to patients on placebo (Repeated Measures Analysis with last observation carried forward [LOCF] for any missing values p=0.015). Results from the analysis of averaged NGPS over time from patients in the 0.2% group compared to placebo was borderline significant(Repeated Measures Analysis with LOCF p=0.054). None of the secondary efficacy variables for clonidine treatment groups were significantly different from placebo. However, weekly averages of NGPS scores from the patient diaries, pain relief scores, and functional interference scores favored the active clonidine formulations.

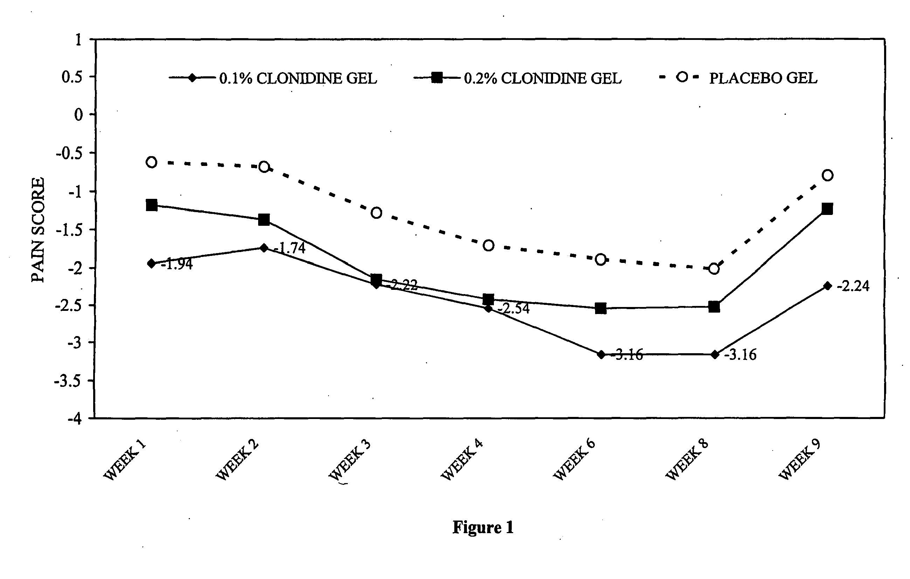

Analgesia from 0.1 % clonidine gel, compared to placebo, was demonstrated as early as the first week of treatment At week 1, the mean change from baseline was -1.87 for the 0.1% clonidine group versus -0.59 for the placebo group (p=0.003). At one week following the discontinuation of treatment (week 9) analgesia continued with a mean change from baseline of -2.43 for the 0.1% clonidine group and -0.95 for the placebo group (p=0.009). The mean change in NGPS from baseline to final visit with the last observation carried forward to account for dropouts or missing visits was -1.96 for placebo, -2.52 for the 0.2% formulation and -2.96 for the 0.1% formulation. Figure 1 shows the mean NGPS reduction by week.

There were no significant intra-patient differences in mean blood pressure or pulse observations, when comparing pre-treatment, during treatment and post-treatment. Likewise there were no significant differences between the three treatment groups. No rebound hypertension was observed following abrupt discontinuation of treatment.

Clonidine plasma concentrations were analyzed in a group of patients who completed the trial. A similar number of samples from a prior trial of 0.05% clonidine were also analyzed at the same time. Table 3 summarizes these results.

**Table 3. Patient clonidine plasma concentrations**

| **Formulation** | **0.05%** | **0.1%** | **0.2%** |
|---|---|---|---|
| Daily Clonidine Dose | 2.5 | 3.9 | 6.0 |
| # Samples | 25 | 24 | 25 |
| # Samples with Measurable Clonidine Concentrations (>0.025 ng/ml) | 13 | 16 | 14 |
| Mean of Measurable Concentrations (ng/ml) | 0.0831 | 0.0749 | 0.0827 |
| Range of Measurable Concentrations (ng/ml) | 0.0290-0.286 | 0.0281-0.176 | 0.0346-0.177 |
| Mean Concentrations for All Samples | 0.0572 | 0.0589 | 0.0583 |

In conclusion, patients with painful diabetic neuropathy on 0.1% clonidine gel had a statistically significantly better analgesic response averaged over time (p=0.015) compared to the response for the placebo group. While the 0.2% clonidine gel was not statistically superior to placebo gel (p=0.054), the trend toward superiority is supportive for topical clonidine analgesia.

Further, plasma clonidine concentrations tended to be far below the threshold levels that are required for a antihypertensive effect (0.2 ng/ml). A lack of significant intra-patient or inter-group changes in blood pressure and a lack or rebound hypertension indicate that topical application of clonidine is relatively free of typical systemic clonidine adverse events.

### Example 3: Single dose pharmacokinetic study with 0.1% clonidine gel

### Materials and Methods

Six volunteers applied single doses of 0.1% clonidine gel up to a maximum of 2 mg clonidine HCl per day. Clonidine plasma concentration analysis was conducted and consisted of a validated gas chromatography/mass spectroscopy method with a quantitative limit of 0.025% ng/ml.

### Results

Clonidine plasma concentration analysis in the group of six volunteers showed all samples to be below the limit of quantitation. Therefore, single doses, even as large as 2.0 mg/day of 0.1 % topical clonidine gel are insufficiently absorbed to have any antihypertensive effect or be of any clinical consequence.

### Example 4: Multiple dose pharmacokinetic study with 0.1% clonidine gel

### Materials and Methods

This investigation was performed as an open-label, randomized, multiple-dose, two-treatment, crossover design study using 8 adult volunteers. The two treatments were: 1) application of 3.15 gm/day (3.1 mg clonidine HCl/day) (treatment A) of 0.1% clonidine gel delivered in three divided doses for 14 days on the right lower leg, and 2) application of 6.23 gm/day (6.2 mg clonidine HCl/day) (treatment B) of 0.1% clonidine gel delivered in three divided doses for 14 days on both lower legs. Treatment lasted for 14 days followed by a 7-day no-treatment observation period. Clonidine plasma concentration analysis was performed as in Example 3.

### Results

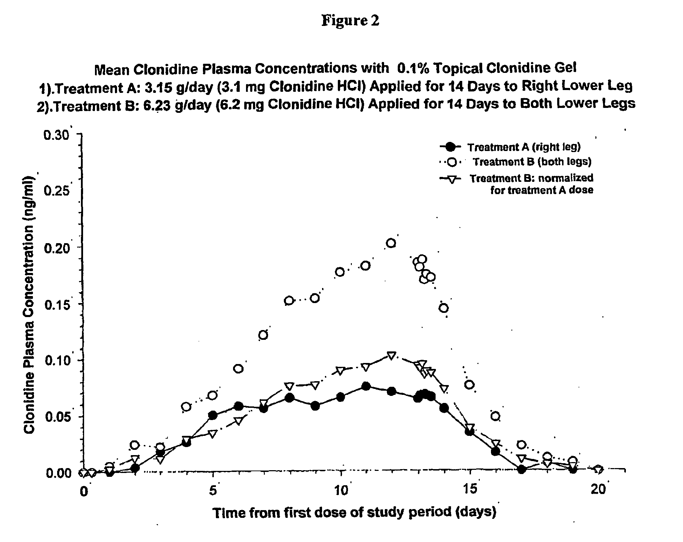

Clonidine plasma concentration data following 14 days of controlled treatment in normal volunteers showed systemic absorption of clonidine after topical application was slow, incomplete, and variable among subjects. After initiation of therapy with the 0.1% topical clonidine gel, a time lag of 48 to 96 hours occurred before systemic absorption of clonidine was observed in the plasma. The mean (CV)t_{lag} was 93.0 hours with treatment A and 78.0 hours with treatment B (p>0.1, A vs. B). After the lag time, clonidine plasma concentrations increased gradually until steady state was achieved between study days 11 and 13. Following the last dose, clonidine plasma concentrations declined with a mean (CV) elimination t_{1/2} of 38.5 hours for treatment A and 35.3 hours for treatment B (p>0.1). It took 7 days before the clonidine concentrations fell below the level of detection. Figure 2 shows a plot of mean plasma concentrations as a function of time.

The apparent dose-dependent pharmacokinetics with topical clonidine gel is of limited clinical importance in view of the substantially lower steady-state plasma concentrations as compared to those reported following clinically applicable doses of oral and transdermal clonidine. The mean Cₘₐₓ of 0.067 ng/ml during treatment A and 0.181 ng/ml during treatment B are both below the threshold of clonidine plasma concentrations associated with the drug's antihypertensive effects (0.2 ng/ml). Additionally, no clinically important changes in blood pressure or heart rate occurred in any subjects during the study.

Unless defined otherwise, all technical and scientific terms used herein have the same meanings as commonly understood by one of skill in the art to which the disclosed invention belongs. Although any methods and materials similar or equivalent to those described herein can be used in the practice or testing of the present invention, the preferred methods, devices, and materials are as described,

Those skilled in the art will recognize, or be able to ascertain using no more than routine experimentation, many equivalents to the specific embodiments of the invention described herein. Such equivalents are intended to be encompassed by the extent of protection as determined by the following claims, which shall be interpreted using the description and drawings.

## Claims

1. An α2 adrenergic agonist compound for use in a method for the treatment of painful length dependent neuropathy in a patient in need thereof, comprising locally or topically administering to or adjacent to painful areas the α2 adrenergic agonist compound in an effective amount at the site of treatment and not systemically, wherein the patient is a patient with length dependent neuropathy that results in pain, which is not sympathetically maintained pain, wherein the compound is clonidine or a pharmaceutically acceptable salt thereof.

2. The α2 adrenergic agonist compound for use according to claim 1 wherein the compound is formulated in a carrier for topical administration.

3. The α2 adrenergic agonist compound for use according to claim 1 wherein 0.01% to 1.0% clonidine is applied to the painful area affected by the neuropathy.

4. The α2 adrenergic agonist compound for use according to claim 1 wherein the clonidine is given from once a week to several times a day.

5. The α2 adrenergic agonist compound for use according to claim 1 wherein the treatment is given to patients with neuropathy that stems from diabetes mellitus.

6. The α2 adrenergic agonist compound for use according to claim 1 wherein the treatment is administered to patients with a sensory peripheral neuropathy in the painful region.

7. The α2 adrenergic agonist compound for use according to claim 1 wherein the treatment is administered to a patient with a small fiber neuropathy in the painful region.

8. The α2 adrenergic agonist compound for use according to claim 1 further comprising administering a therapy selected from the group consisting of opioids, anticonvulsants, membrane stabilizers, and psychoactive drugs such as anti-depressants.

9. A dosage formulation for use in a method for the treatment of painful length dependent neuropathy in a patient in need thereof,
the dosage formulation comprising an α2 adrenergic agonist compound in a pharmaceutically acceptable carrier for local or topical administration,
the method comprising locally or topically administering to or adjacent to painful areas the α2 adrenergic agonist compound in an effective amount at the site of treatment and not systemically,
wherein the patient is a patient with length dependent neuropathy that results in pain, which is not sympathetically maintained pain,
wherein the α2 adrenergic agonist compound is clonidine or a pharmaceutically acceptable salt thereof.

10. The dosage formulation for use according to claim 9, wherein the carrier is selected from the group of transdermal patches, gels, ointments, lotions, liposomal formulations, creams and emulsions.

11. The dosage formulation for use according to claim 9, wherein the clonidine is in a concentration between 0.1 and 10% clonidine.

12. The dosage formulation for use according to claim 9 comprising 0.1% to 1.0% clonidine.

13. The dosage formulation for use according to claim 9 further comprising a therapeutic selected from the group consisting of opioids, anticonvulsants, membrane stabilizers, and psychoactive drugs.

14. The α2 adrenergic agonist compound for use according to claim 1, wherein the compound is formulated in a topical carrier selected from the group consisting of transdermal patches, gels, ointments, lotions, liposomal formulations, creams and emulsions.

## Patentansprüche

1. α2-adrenerge Agonistverbindung zur Verwendung in einem Verfahren zur Behandlung von schmerzhafter, längenabhängiger Neuropathie bei einem Patienten, der dessen bedarf, welches lokale oder oberflächliche Verabreichung der α2-adrenergen Agonistverbindung in einer wirksamen Menge an der Behandlungsstelle in oder neben die schmerzhaften Bereiche umfasst, jedoch nicht systematisch, wobei der Patient ein Patient mit einer längenabhängigen Neuropathie ist, die in Schmerz resultiert, der jedoch kein Sympathikus-abhängiger Schmerz ist, wobei die Verbindung ein Clonidin oder ein pharmazeutisch akzeptables Salz davon ist.

2. α2-adrenerge Agonistverbindung zur Verwendung gemäss Anspruch 1, wobei die Verbindung in einem Träger zur oberflächlichen Verabreichung formuliert ist.

3. α2-adrenerge Agonistverbindung zur Verwendung gemäss Anspruch 1, wobei 0,01 bis 1,0 % Clonidin bei dem von der Neuropathie betroffenen schmerzhaften Bereich eingesetzt wird.

4. α2-adrenerge Agonistverbindung zur Verwendung gemäss Anspruch 1, wobei das Clonidin von einmal wöchentlich bis mehrmals täglich verabreicht wird.

5. α2-adrenerge Agonistverbindung zur Verwendung gemäss Anspruch 1, wobei die Behandlung Patienten mit Neuropathie verabreicht wird, die von Diabetes mellitus abstammt.

6. α2-adrenerge Agonistverbindung zur Verwendung gemäss Anspruch 1, wobei die Behandlung bei Patienten mit einer sensorischen peripheren Neuropathie in den schmerzhaften Bereich verabreicht wird.

7. α2-adrenerge Agonistverbindung zur Verwendung gemäss Anspruch 1, wobei die Behandlung bei Patienten mit einer Small Fiber-Neuropathie in den schmerzhaften Bereich verabreicht wird.

8. α2-adrenerge Agonistverbindung zur Verwendung gemäss Anspruch 1, welche weiterhin die Verabreichung einer Therapie, ausgewählt aus Opiaten, Antikonvulsanten, Membranstabilisatoren und psychoaktiven Wirkstoffen, wie Antidepressiva, umfasst.

9. Dosisformulierung zur Verwendung in einem Verfahren zur Behandlung von schmerzhafter, längenabhängiger Neuropathie bei einem Patienten der dessen bedarf,
die Dosisformulierung umfasst eine α2-adrenerge Agonistverbindung in einem pharmazeutisch akzeptablen Träger zur lokalen oder oberflächlichen Verabreichung,
das Verfahren umfasst lokale oder oberflächliche Verabreichung der α2-adrenergen Agonistverbindung in einer wirksamen Menge an der Behandlungsstelle in oder neben die schmerzhaften Bereiche, jedoch nicht systematisch,
wobei der Patient ein Patient mit einer längenabhängigen Neuropathie ist, der in Schmerz resultiert, der jedoch kein Sympathikus-abhängiger Schmerz ist,
wobei die α2-adrenerge Agonistverbindung ein Clonidin oder ein pharmazeutisch akzeptables Salz davon ist.

10. Dosisformulierung zur Verwendung gemäss Anspruch 9, wobei der Träger aus transdermalen Pflastern, Gelen, Salben, Lotionen, liposomalen Formulierungen, Cremes und Emulsionen ausgewählt ist.

11. Dosisformulierung zur Verwendung gemäss Anspruch 9, wobei das Clonidin in einer Konzentration zwischen 0,1 und 10 % Clonidin vorliegt.

12. Dosisformulierung zur Verwendung gemäss Anspruch 9, die 0,1 bis 1,0 % Clonidin umfasst.

13. Dosisformulierung zur Verwendung gemäss Anspruch 9, die weiterhin ein Therapeutikum, ausgewählt aus Opiaten, Antikonvulsanten, Membranstabilisatoren und psychoaktiven Wirkstoffen, umfasst.

14. α2-adrenerge Agonistverbindung zur Verwendung gemäss Anspruch 1, wobei die Verbindung in einem topischen Träger formuliert ist, der aus transdermalen Pflastern, Gelen, Salben, Lotionen, liposomalen Formulierungen, Cremes und Emulsionen ausgewählt ist.

## Revendications

1. Composé agoniste α2 adrénergique pour une utilisation dans un procédé destiné au traitement d'une neuropathie douloureuse longueur-dépendante chez un patient en ayant besoin, comprenant l'administration locale ou topique sur, ou au voisinage, des zones douloureuses du composé agoniste α2 adrénergique dans une quantité efficace au niveau du site de traitement et non par voie systémique, dans lequel le patient est un patient avec une neuropathie longueur-dépendante qui entraîne une douleur, laquelle n'est pas une douleur maintenue par voie sympathique,
dans lequel le composé est la clonidine ou un sel pharmaceutiquement acceptable de celle-ci.

2. Composé agoniste α2 adrénergique pour une utilisation selon la revendication 1, dans lequel le composé est formulé dans un véhicule pour une administration topique.

3. Composé agoniste α2 adrénergique pour une utilisation selon la revendication 1, dans lequel 0,01 % à 1,0 % de clonidine est appliqué sur la zone douloureuse touchée par la neuropathie.

4. Composé agoniste α2 adrénergique pour une utilisation selon la revendication 1, dans lequel la clonidine est donnée de une fois par semaine à plusieurs fois par jour.

5. Composé agoniste α2 adrénergique pour une utilisation selon la revendication 1, dans lequel le traitement est donné à des patients avec une neuropathie qui résulte d'un diabète sucré.

6. Composé agoniste α2 adrénergique pour une utilisation selon la revendication 1, dans lequel le traitement est administré à des patients avec une neuropathie sensitive périphérique dans la région douloureuse.

7. Composé agoniste α2 adrénergique pour une utilisation selon la revendication 1, dans lequel le traitement est administré à un patient avec une neuropathie des petites fibres dans la région douloureuse.

8. Composé agoniste α2 adrénergique pour une utilisation selon la revendication 1, comprenant en outre l'administration d'une thérapie choisie dans le groupe constitué par les opiacés, les anticonvulsivants, les stabilisants membranaires, et les médicaments psychotropes tels que les antidépresseurs.

9. Formulation posologique pour une utilisation dans un procédé destiné au traitement d'une neuropathie douloureuse longueur-dépendante chez un patient en ayant besoin,
la formulation posologique comprenant un composé agoniste α2 adrénergique dans un véhicule pharmaceutiquement acceptable pour une administration locale ou topique,
le procédé comprenant l'administration locale ou topique sur, ou au voisinage, des zones douloureuses du composé agoniste α2 adrénergique dans une quantité efficace au niveau du site de traitement et non par voie systémique,
dans laquelle le patient est un patient avec une neuropathie longueur-dépendante qui entraîne une douleur, laquelle n'est pas une douleur maintenue par voie sympathique,
dans laquelle le composé agoniste α2 adrénergique est la clonidine ou un sel pharmaceutiquement acceptable de celle-ci.

10. Formulation posologique pour une utilisation selon la revendication 9, dans laquelle le véhicule est choisi dans le groupe des timbres transdermiques, des gels, des pommades, des lotions, des formulations liposomales, des crèmes et des émulsions.

11. Formulation posologique pour une utilisation selon la revendication 9, dans laquelle la clonidine est à une concentration entre 0,1 et 10 % de clonidine.

12. Formulation posologique pour une utilisation selon la revendication 9, comprenant 0,1 % à 1,0 % de clonidine.

13. Formulation posologique pour une utilisation selon la revendication 9, comprenant en outre un agent thérapeutique choisi dans le groupe constitué par les opiacés, les anticonvulsivants, les stabilisants membranaires, et les médicaments psychotropes

14. Composé agoniste α2 adrénergique pour une utilisation selon la revendication 1, dans lequel le composé est formulé dans un véhicule topique choisi dans le groupe constitué par les timbres transdermiques, les gels, les pommades, les lotions, les formulations liposomales, les crèmes et les émulsions.
